# EUROPÄISCHE PATENTANMELDUNG

(11) **EP 4 116 700 A2**
(43) Veröffentlichungstag der Anmeldung: **11.01.2023**
(21) Anmeldenummer: 22182835.3
(22) Anmeldetag: 04.07.2022
(51) Int. Cl.: G01N 21/01, B01L 3/00, G01N 27/48, G01N 27/00, G01N 21/80, G01N 21/03, G01N 21/77

(54) **ADAPTER, FUNKTIONSEINHEIT UND VERFAHREN**

(30) Priorität: 05.07.2021 LU 102839
(71) Anmelder: Eppendorf SE, 22339 Hamburg (DE)
(72) Erfinder: KOCH, Laura Sophie, 22889 Tangstedt (DE); OESER, Alexander, 22307 Hamburg (DE); SEFEKE, Steffen, 22089 Hamburg (DE)
(74) Vertreter: Eisenführ Speiser

(57) **Zusammenfassung**

Adapter (100) zur Fixierung eines an einer stirnseitigen Auflagefläche (111) eines Trägers (110) für eine Sensoreinrichtung angeordneten flächigen membranartigen Funktionselements, umfassend einen Halterahmen (130), der ausgebildet ist, um einen stirnseitigen Endbereich (112) des Trägers (110) zumindest teilweise zu umgeben, einen beweglich mit dem Halterahmen verbundenen Verriegelungsrahmen (140), der zumindest teilweise eine Funktionsöffnung (141) umgibt und ausgebildet ist, um das flächige membranartige Funktionselement (120) in einer Schließstellung an der stirnseitigen Auflagefläche (111) mechanisch zu fixieren, wobei der Verriegelungsrahmen (140) ausgebildet ist, um formschlüssig an dem Halterahmen (130) in der Schließstellung arretiert zu werden.

## Beschreibung

Die Erfindung betrifft einen Adapter zur Fixierung eines flächigen Funktionselementes, eine Sensoreinrichtung, ein Verfahren zur Herstellung eines Adapters sowie ein Verfahren zur Fixierung.

Verschiedene Anwendungen, insbesondere in Bioreaktoren, erfordern eine Fixierung flächiger, zumeist membranartiger, Funktionselemente an einem Träger. So müssen beispielsweise optochemische Sensorspots für die nicht-invasive Messung des pH-Werts in Bioreaktoren auf Trägern für Sensoreinrichtungen befestigt werden, um den pH-Wert bestimmen zu können.

In bekannten Systemen werden derartige Funktionselemente, wie beispielsweise die Membranen derartiger optischer pH-Sensoren oder Filter zum Begasen oder zur Probenentnahme aufgeklebt. Derartige Klebungen haben zum Teil hohe Ausfallraten und können durch Lösen des Klebers im umgebenden Milieu zusätzlich Messergebnisse verfälschen und Proben oder Kulturen kontaminieren.

Der Erfindung liegt daher die Aufgabe zugrunde, eine verbesserte Lösung bereitzustellen, welche die genannten Probleme adressiert. Insbesondere ist es eine Aufgabe der Erfindung, eine Lösung bereitzustellen, die ein Ablösen des Funktionselementes zuverlässig verhindert.

Gemäß einem ersten Aspekt betrifft die Erfindung einen Adapter zur Fixierung eines an einer stirnseitigen Auflagefläche eines Trägers für eine Sensoreinrichtung angeordneten flächigen membranartigen Funktionselements umfassend
- einen Halterahmen, der ausgebildet ist, um einen stirnseitigen Endbereich des Trägers zumindest teilweise zu umgeben,
- einen beweglich mit dem Halterahmen verbundenen Verriegelungsrahmen, der zumindest teilweise eine Funktionsöffnung umgibt und ausgebildet ist, um das flächige membranartige Funktionselement in einer Schließstellung an der stirnseitigen Auflagefläche mechanisch zu fixieren, vorzugsweise zu verklemmen.
- wobei der Verriegelungsrahmen ausgebildet ist, um formschlüssig an dem Halterahmen in der Schließstellung arretiert zu werden.

Der Erfindung liegt unter anderem die Erkenntnis zugrunde, dass die bisher verwendeten Klebungen vorteilhaft durch eine mechanische Fixierung ersetzt werden können und dass insbesondere ein Kombination aus Halterahmen am Träger und mit diesem beweglich verbundenem Verriegelungsrahmen eine zugleich einfach handhabbare und sichere Fixierung erlaubt. Der erfindungsgemäße Adapter erlaubt eine präzise Positionierung des Funktionselementes am Träger, die dann vom beweglichen Verriegelungsrahmen fixiert wird. Kontaminationen durch Klebstoffe oder ähnliches Entfallen und die Fixierung erlaubt ein sicheres und einfaches Handling des Funktionselementes. Darüber hinaus stellt die bewegliche Verbindung von Halteelement und Verriegelungselement sicher, dass letzteres auch bei einem Wechsel des Funktionselementes am Halteelement verbleibt und nicht verloren gehen oder herunterfallen kann. Die Sensoreinrichtung kann insbesondere eine Sensoreinheit oder eine Sensorkappe sein. Die Erfindung ist insbesondere vorteilhaft bei einem optochemischen oder elektrochemischen Sensor als Sensoreinheit einsetzbar, da gerade hier Kontaminationen durch Klebstoffe besonders störend sind. Das membranartige Funktionselement kann insbesondere ein flexibles Folienelement oder eine Membran sein.

Nachfolgend werden Ausführungsbeispiele des erfindungsgemäßen Adapters beschrieben.

In einer Ausführungsform umfasst der Adapter zumindest ein sich im Wesentlichen radial in die Funktionsöffnung erstreckendes Klemmelement, das ausgebildet ist, um das flächige membranartige Funktionselement an der stirnseitigen Auflagefläche mechanisch zu fixieren, wobei vorzugsweise das zumindest eine Klemmelement, insbesondere ein Federelement oder Klemmfinger ist und, weiter bevorzugt, aus einer Montagestellung, in der das zumindest eine Klemmelement einen ersten Durchmesser begrenzt, in die Schließstellung, in der das zumindest eine Klemmelement einen zweiten Durchmesser begrenzt, axial beweglich ist, wobei der erste Durchmesser kleiner als der zweite Durchmesser ist. Ein derartiges Klemmelement stellt eine besonders einfach handhabbare und sichere Fixierung bereit. Bevorzugt umfasst der Adapter genau drei Klemmelemente.

In einer weiteren Ausführungsform umfasst der Adapter den Träger, der die stirnseitige Auflagefläche aufweist, wobei der Halterahmen an dem stirnseitigen Endbereich des Trägers derart angeordnet ist, dass dieser den stirnseitigen Endbereich des Trägers zumindest teilweise umgibt. Dabei ist vorzugsweise der Halterahmen mit dem Trägerformschlüssig und/oder kraftschlüssig und/oder stoffschlüssig verbunden oder der Halterahmen und der Träger sind integral hergestellt oder der Halterahmen ist an den Träger integral angeformt. Die Anordnung umgebend den Endbereich des Trägers erlaubt eine besonders verrutschsichere Ausführung. Der Halterahmen kann insbesondere auch an den Träger angespritzt sein. Der Träger ist bevorzugt zylinderförmig oder als Prisma, bevorzugt als gerades Prisma ausgebildet. Der Träger ist als Träger für eine Sensoreinrichtung ausgebildet, also bevorzugt ausgebildet, diese aufzunehmen oder auf ihr angeordnet zu werden und mithin bevorzugt teilweise hohl.

In einer weiteren Ausführungsform weist der Verriegelungsrahmen einen Verriegelungshaken und der Halterahmen einen in der Schließstellung vom Verriegelungshaken hintergriffenen Verriegelungsnocken auf. Hierüber kann der Verriegelungsrahmen vorteilhaft am Halterahmen verriegelt werden und ist damit gegen ungewolltes Öffnen noch besser geschützt.

Der Adapter umfasst in einer weiteren Ausführungsform eine Gelenkverbindung, die den Halterahmen beweglich mit dem Verriegelungsrahmen verbindet, wobei vorzugsweise die Gelenkverbindung ein Filmscharnier ist. Bevorzugt sind dabei der Halterahmen und die Gelenkverbindung und/oder der Verriegelungsrahmen und die Gelenkverbindung integral ausgebildet und/oder stoffschlüssig miteinander verbunden und somit gegen Lösen voneinander besser geschützt.

Der Adapter umfasst bevorzugt Kunststoff und/oder Metall, insbesondere Stahl, umfasst oder ist zumindest teilweise aus Kunststoff oder Metall, insbesondere Stahl, hergestellt.

Der Adapter ist in weiteren Ausführungsformen zumindest teilweise in einem additiven Herstellungsverfahren und/oder Spritzgussverfahren hergestellt ist.

Der Adapter kann weiter ausgebildet sein, insbesondere über den Halterahmen oder Träger formschlüssig und/oder kraftschlüssig und/oder stoffschlüssig an der Sensoreinrichtung befestigt zu werden. Ein form- und/oder kraftschlüssige Verbindung bietet den Vorteil eines festen Sitzes auf der Sensoreinrichtung bei gleichzeitiger Lösbarkeit ohne Zerstörung der Partner, so dass der Adapter auch für wechselnde Sensoreinrichtungen verwendet werden kann.

In einer Ausführungsform umfasst der Träger ein Außengewinde zum einfachen Einbringen und Fixieren des Trägers in einer Kopfplatte eines Bioreaktors. Alternativ kann der Träger auch als integraler Bestandteil einer Kopfplatte eines Bioreaktors ausgeführt sein oder in einer solchen über eine Klemm- oder sonstige Fixiervorrichtung befestigbar sein.

Gemäß einem zweiten Aspekt umfasst die Sensoreinrichtung einen Grundkörper und einen an dem Grundkörper angeordneten Adapter, insbesondere gemäß dem ersten Aspekt der Erfindung, mit
- einem Halterahmen,
- einem beweglich mit dem Halterahmen verbundenen Verriegelungsrahmen, der zumindest teilweise eine Funktionsöffnung umgibt und ausgebildet ist, um formschlüssig an dem Halterahmen in einer Schließstellung arretiert zu werden,
- einem an dem Grundkörper angeordneten Träger, der eine stirnseitige Auflagefläche aufweist, wobei der Halterahmen an dem stirnseitigen Endbereich des Trägers derart angeordnet ist, dass dieser den stirnseitigen Endbereich des Trägers zumindest teilweise umgibt, und
- einem flächigen Funktionselement, das an der stirnseitigen Auflagefläche angeordnet ist,
- wobei der Verriegelungsrahmen des Adapters ausgebildet ist, um das flächige membranartige Funktionselement in einer Schließstellung an der stirnseitigen Auflagefläche mechanisch zu fixieren,
- wobei der Verriegelungsrahmen formschlüssig an dem Halterahmen in der Schließstellung arretiert ist, sodass das flächige membranartige Funktionselement an der stirnseitigen Auflagefläche mechanisch fixiert ist.

In einer Ausführungsform umfasst die Sensoreinrichtung eine Sensoreinheit, insbesondere einen optochemischen Sensor oder einen elektrochemischen Sensor zur Bestimmung und/oder Überwachung einer Messgröße eines Messmediums, mit dem Grundkörper oder eine Sensorkappe zum, insbesondere lösbaren, Verbinden an eine Sensoreinheit mit dem Grundkörper.

In einer weiteren Ausführungsform ist der Adapter formschlüssig und/oder kraftschlüssig und/oder stoffschlüssig mit dem Grundkörper verbunden oder integral mit dem Grundkörper ausgebildet. Der Adapter kann insbesondere lösbar mit dem Grundkörper verbunden sein, so dass ein Auswechseln erleichtert wird.

Das flächige membranartige Funktionselement ist bevorzugt eine Membran für eine Sensoreinheit, insbesondere für einen optochemischen Sensor oder einen elektrochemischen Sensor, zur Bestimmung und/oder Überwachung einer Messgröße eines Messmediums. Die Membran ist vorzugsweise zumindest teilweise in Form einer porösen, insbesondere Poren aufweisenden, Struktur ausgestaltet. Die Membran ist weiter vorzugsweise zumindest teilweise als eine Folie ausgestaltet.

Gemäß einem dritten Aspekt betrifft die Erfindung ein Verfahren zum Herstellen eines Adapters, insbesondere eines Adapters gemäß dem ersten Aspekt der Erfindung, umfassend Spritzgießen und/oder additives Fertigen zumindest eines Teils eines Adapters. Das Verfahren umfasst vorzugsweise die Schritte
- Spritzgießen oder additives Fertigen eines Halterahmens und/oder
- Spritzgießen oder additives Fertigen eines Verriegelungsrahmens und/oder
- Verbinden des Halterahmens mit dem Verriegelungsrahmen derart, dass diese beweglich miteinander verbunden sind, beispielsweise mittels einer Gelenkverbindung, und/oder
- Spritzgießen oder additives Fertigen eines Halterahmens, eines Verriegelungsrahmens und eines Filmscharniers zwischen dem Halterahmen und dem Verriegelungsrahmen als einstückiges Bauteil und/oder
- Spritzgießen oder additives Fertigen eines Trägers und/oder
- formschlüssiges und/oder kraftschlüssiges und/oder stoffschlüssiges Verbinden des Halterahmens mit dem Träger und/oder
- integrales Herstellen des Halterahmens und des Trägers und/oder
- integrales Anformen des Halterahmens an dem Träger.

Insbesondere kann der Halterahmen spritzgegossen oder additiv gefertigt und die Gelenkverbindung und der Verriegelungsrahmen an den Halterahmen angespritzt werden. Alternativ kann auch Verriegelungsrahmen spritzgegossen oder additiv gefertigt und die Gelenkverbindung und der Halterahmen an den Verriegelungsrahmen angespritzt werden.

Gemäß einem vierten Aspekt betrifft die Erfindung ein Verfahren zum Fixieren eines flächigen Funktionselements mit einem Adapter, insbesondere einem Adapter gemäß dem ersten Aspekt der Erfindung, umfassend die Schritte
- Anordnen eines flächigen Funktionselements an einer stirnseitigen Auflagefläche eines Trägers,
- Mechanisches Fixieren des flächigen Funktionselements an der stirnseitigen Auflagefläche des Trägers durch Bewegen eines beweglich mit einem einen stirnseitigen Endbereich des Trägers zumindest teilweise umgebenden Halterahmen verbundenen Verriegelungsrahmens des Adapters in eine Schließstellung und formschlüssiges Arretieren des Verriegelungsrahmens an dem Halterahmen in der Schließstellung.

In einer Ausführungsform wird das flächige membranartige Funktionselement vor dem Anordnen ausgestanzt. Dies ist insbesondere bevorzugt, wenn das flächige membranartige Funktionselement eine Membran ist.

In weiteren Ausführungsformen kann der Adapter in weiteren Schritten in einer Kopfplatte angeordnet werden, beispielsweise durch Eindrehen, wenn ein Außengewinde am Träger vorhanden ist oder durch Einstecken. Dabei ist es auch vorteilhaft, wenn ein zusätzlicher Dichtring zur Abdichtung verwendet wird.

Mögliche Ausführungen und Vorteile, welche mit Bezug auf den Adapter beschrieben sind, beziehen sich ebenso auf die Sensoreinrichtung sowie die Verfahren. So können auch für die Sensoreinrichtung beliebige Ausführungsformen und Weiterbildungen des Adapters, wie zuvor erläutert, verwendet werden. Für weitere Vorteile, Ausführungsvarianten und Ausführungsdetails dieser weiteren Aspekte und ihrer möglichen Fortbildungen wird daher auch auf die zuvor erfolgte Beschreibung zu den entsprechenden Merkmalen und Fortbildungen des Adapters verwiesen.

Bevorzugte Ausführungsformen der Erfindung werden beispielhaft anhand der beiliegenden Figuren erläutert. Es zeigen:
Fig. 1 eine schematische Darstellung einer Ausführungsform eines Adapters gemäß dem ersten Aspekt der Erfindung in einer geöffneten Stellung;
Fig. 2 eine schematische Darstellung der Ausführungsform eines Adapters aus Fig. 1 in einer Schließstellung;
Fig. 3 eine schematische Darstellung einer Ausführungsform einer Sensoreinrichtung mit einem Adapter.

In der nachfolgenden Beschreibung von Ausführungsbeispielen beziehen sich ähnliche Bezugszeichen in der Regel auf ähnliche Elemente.

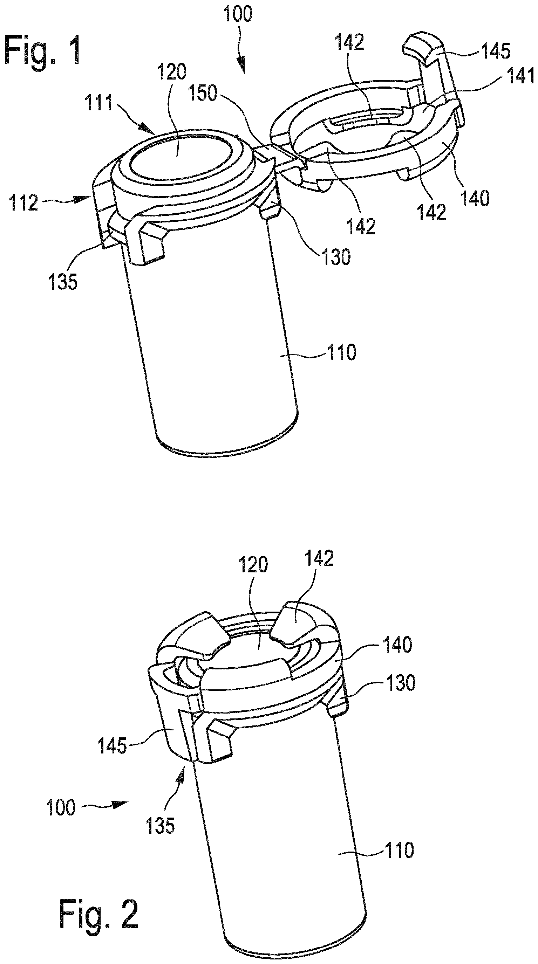

Fig. 1 zeigt eine schematische Darstellung einer Ausführungsform eines Adapters 100 gemäß dem ersten Aspekt der Erfindung in einer geöffneten Position. Der Adapter 100 zum Anordnen an einer Sensoreinrichtung zur Fixierung eines an einer stirnseitigen Auflagefläche 111 eines Trägers 110 angeordneten flächigen membranartigen Funktionselements umfasst in der gezeigten Ausführungsform, einen Träger 110, einen am Träger 110 angeordneten Halterahmen 130 und einen über eine Gelenkverbindung 150 mit dem Halterahmen 130 verbundenen Verriegelungsrahmen 140. Der Halterahmen 130 ist ausgebildet, um einen stirnseitigen Endbereich 112 des Trägers 110 zu umgeben. Der Verriegelungsrahmen 140 umgibt eine Funktionsöffnung 141 und ist, um das flächige membranartige Funktionselement 120, hier eine Membran, in einer Schließstellung an der stirnseitigen Auflagefläche 111 mechanisch zu fixieren. Die Schließstellung wird nachfolgend in Bezug auf Fig. 2 näher erläutert. Der Verriegelungsrahmen 140 ist weiter ausgebildet, um formschlüssig an dem Halterahmen 130 in der Schließstellung arretiert zu werden. Der Verriegelungsrahmen 140 weist in der gezeigten Ausführungsform einen Verriegelungshaken 145 auf und der Halterahmen weist einen Verriegelungsnocken 135 auf. Über Verriegelungshaken 145 und Verriegelungsnocken 135 wird der Verriegelungsrahmen 140 am Halterahmen 130 in der Schließstellung arretiert. Der Adapter 100 ist hier in einer geöffneten Position zu sehen. Der Verriegelungsrahmen 140 ist über die Gelenkverbindung 150, die hier als Filmscharnier ausgebildet ist, vom Träger weggeklappt, so dass das flächige membranartige Funktionselement 120, hier die Membran, auf die stirnseitige Auflagefläche 111 des Trägers 110 aufgelegt werden kann. Am Verriegelungsrahmen 140 sind in der gezeigten Ausführungsform drei sich im Wesentlichen radial in die Funktionsöffnung 141 erstreckende Klemmelemente 142 angeordnet. Diese sind ausgebildet, um das flächige membranartige Funktionselement 120 an der stirnseitigen Auflagefläche 111 mechanisch zu fixieren. Der Halterahmen 130 ist hier an dem stirnseitigen Endbereich 112 des Trägers derart angeordnet ist, dass er den stirnseitigen Endbereich des Trägers umgibt.

Fig. 2 zeigt eine schematische Darstellung der Ausführungsform eines Adapters aus Fig. 1 in einer Schließstellung. In der gezeigten Schließstellung wird der Verriegelungsnocken 135 vom Verriegelungshaken hintergriffen und so der Verriegelungsrahmen 140 am Halterahmen arretiert und verriegelt. Gleichzeitig fixieren die Klemmelemente 142 das flächige membranartige Funktionselement 120 am Träger 110 und verhindern so ein Verrutschen oder Ablösen.

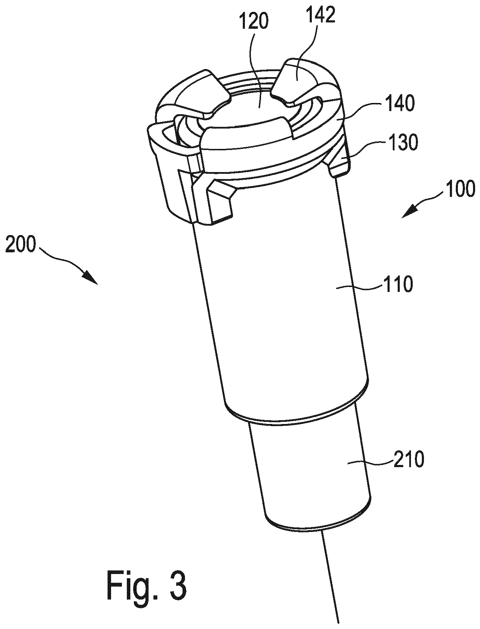

Fig. 3 zeigt eine schematische Darstellung einer Ausführungsform einer Sensoreinrichtung 200 mit einem Adapter 100. Die Sensoreinrichtung 200 umfasst einen Grundkörper 210 und einen an dem Grundkörper 210 angeordneten Adapter 100 mit einem Halterahmen 130, einem beweglich mit dem Halterahmen verbundenen Verriegelungsrahmen 140, der eine Funktionsöffnung umgibt und ausgebildet ist, um formschlüssig an dem Halterahmen 130 in der hier gezeigten Schließstellung arretiert zu werden. Der Adapter 100 weist außerdem einen an dem Grundkörper 210 angeordneten Träger 110 auf, der eine stirnseitige Auflagefläche aufweist, wobei der Halterahmen 130 an dem stirnseitigen Endbereich des Trägers derart angeordnet ist, dass dieser den stirnseitigen Endbereich des Trägers zumindest teilweise umgibt, und ein flächiges Funktionselement 120, das an der stirnseitigen Auflagefläche angeordnet ist und von drei sich im Wesentlichen radial in die Funktionsöffnung 141 erstreckenden Klemmelementen 142 an der stirnseitigen Auflagefläche mechanisch fixiert wird. Der Verriegelungsrahmen 140 ist formschlüssig an dem Halterahmen in der Schließstellung befestigt, sodass das flächige membranartige Funktionselement an der stirnseitigen Auflagefläche mechanisch fixiert ist. Der Grundkörper 210 ist hier ein Grundkörper eines optochemischen Sensors, der in den Träger eingesteckt und mit diesem formschlüssig verbunden ist.

### Bezuqszeichenliste

- 100: Adapter
- 110: Träger
- 111: Auflagefläche
- 112: Endbereich
- 120: Funktionselement
- 130: Halterahmen
- 135: Verriegelungsnocken
- 140: Verriegelungsrahmen
- 141: Funktionsöffnung
- 142: Klemmelement
- 145: Verriegelungshaken
- 150: Gelenkverbindung
- 200: Sensoreinrichtung
- 210: Grundkörper

## Patentansprüche

1. Adapter (100) zur Fixierung eines an einer stirnseitigen Auflagefläche (111) eines Trägers (110) für eine Sensoreinrichtung angeordneten flächigen membranartigen Funktionselements, umfassend
einen Halterahmen (130), der ausgebildet ist, um einen stirnseitigen Endbereich (112) des Trägers (110) zumindest teilweise zu umgeben,
einen beweglich mit dem Halterahmen verbundenen Verriegelungsrahmen (140), der zumindest teilweise eine Funktionsöffnung (141) umgibt und ausgebildet ist, um das flächige membranartige Funktionselement (120) in einer Schließstellung an der stirnseitigen Auflagefläche (111) mechanisch zu fixieren ,
wobei der Verriegelungsrahmen (140) ausgebildet ist, um formschlüssig an dem Halterahmen (130) in der Schließstellung arretiert zu werden.

2. Adapter (100) nach dem vorhergehenden Anspruch 1, umfassend zumindest ein sich im Wesentlichen radial in die Funktionsöffnung (141) erstreckendes Klemmelement (142), das ausgebildet ist, um das flächige membranartige Funktionselement (120) an der stirnseitigen Auflagefläche (111) mechanisch zu fixieren.

3. Adapter nach mindestens einem der vorhergehenden Ansprüche, umfassend den Träger, der die stirnseitige Auflagefläche aufweist, wobei der Halterahmen an dem stirnseitigen Endbereich des Trägers derart angeordnet ist, dass dieser den stirnseitigen Endbereich des Trägers zumindest teilweise umgibt.

4. Adapter nach mindestens einem der vorhergehenden Ansprüche, wobei der Verriegelungsrahmen einen Verriegelungshaken (145) und der Halterahmen einen in der Schließstellung vom Verriegelungshaken hintergriffenen Verriegelungsnocken (135) aufweist.

5. Adapter nach mindestens einem der vorhergehenden Ansprüche, umfassend eine Gelenkverbindung (150), die den Halterahmen beweglich mit dem Verriegelungsrahmen verbindet, wobei vorzugsweise die Gelenkverbindung ein Filmscharnier ist.

6. Adapter nach mindestens dem vorhergehenden Anspruch 5, wobei der Halterahmen und die Gelenkverbindung und/oder der Verriegelungsrahmen und die Gelenkverbindung integral ausgebildet und/oder stoffschlüssig miteinander verbunden sind.

7. Adapter nach mindestens einem der vorhergehenden Ansprüche, wobei der Adapter Kunststoff und/oder Metall, insbesondere Stahl, umfasst oder zumindest teilweise aus Kunststoff oder Metall, insbesondere Stahl, hergestellt ist.

8. Adapter nach mindestens einem der vorhergehenden Ansprüche, wobei der Adapter zumindest teilweise in einem additiven Herstellungsverfahren und/oder Spritzgussverfahren hergestellt ist.

9. Adapter nach mindestens einem der vorhergehenden Ansprüche, wobei der Adapter ausgebildet ist, um formschlüssig und/oder kraftschlüssig und/oder stoffschlüssig an der Sensoreinrichtung befestigt zu werden.

10. Sensoreinrichtung (200) umfassend einen Grundkörper (210) und einen an dem Grundkörper angeordneten Adapter, insbesondere nach einem der vorhergehenden Ansprüche 1 bis 9, mit
- einem Halterahmen,
- einem beweglich mit dem Halterahmen verbundenen Verriegelungsrahmen, der zumindest teilweise eine Funktionsöffnung umgibt und ausgebildet ist, um formschlüssig an dem Halterahmen in einer Schließstellung arretiert zu werden,
- einem an dem Grundkörper angeordneten Träger, der eine stirnseitige Auflagefläche aufweist, wobei der Halterahmen an dem stirnseitigen Endbereich des Trägers derart angeordnet ist, dass dieser den stirnseitigen Endbereich des Trägers zumindest teilweise umgibt, und
- einem flächigen Funktionselement, das an der stirnseitigen Auflagefläche angeordnet ist,
wobei der Verriegelungsrahmen des Adapters ausgebildet ist, um das flächige membranartige Funktionselement in einer Schließstellung an der stirnseitigen Auflagefläche mechanisch zu fixieren,
wobei der Verriegelungsrahmen formschlüssig an dem Halterahmen in der Schließstellung befestigt ist, sodass das flächige membranartige Funktionselement an der stirnseitigen Auflagefläche mechanisch fixiert ist.

11. Sensoreinrichtung nach dem vorhergehenden Anspruch 10, umfassend eine Sensoreinheit, insbesondere einen optochemischen Sensor oder einen elektrochemischen Sensor zur Bestimmung und/oder Überwachung einer Messgröße eines Messmediums, mit dem Grundkörper oder eine Sensorkappe zum, insbesondere lösbaren, Verbinden an eine Sensoreinheit mit dem Grundkörper.

12. Sensoreinrichtung nach mindestens dem einem der vorhergehenden Ansprüche 10 oder 11, wobei der Adapter formschlüssig und/oder kraftschlüssig und/oder stoffschlüssig mit dem Grundkörper verbunden ist oder integral mit dem Grundkörper ausgebildet ist.

13. Sensoreinrichtung nach mindestens einem der vorhergehenden Ansprüche 10 bis 12, wobei das flächige membranartige Funktionselement eine Membran für eine Sensoreinheit, insbesondere einen optochemischen Sensor oder einen elektrochemischen Sensor, zur Bestimmung und/oder Überwachung einer Messgröße eines Messmediums ist.

14. Verfahren zum Herstellen eines Adapters, insbesondere nach einem der Ansprüche 1 bis 9, umfassend Spritzgießen und/oder additives Fertigen zumindest eines Teils eines Adapters, vorzugsweise umfassend die Schritte
- Spritzgießen oder additives Fertigen eines Halterahmens und/oder
- Spritzgießen oder additives Fertigen eines Verriegelungsrahmens und/oder
- Verbinden des Halterahmens mit dem Verriegelungsrahmen derart, dass diese beweglich miteinander verbunden sind, und/oder
- Spritzgießen oder additives Fertigen eines Halterahmens, eines Verriegelungsrahmens und eines Filmscharniers zwischen dem Halterahmen und dem Verriegelungsrahmen als einstückiges Bauteil und/oder
- Spritzgießen oder additives Fertigen eines Trägers und/oder
- formschlüssiges und/oder kraftschlüssiges und/oder stoffschlüssiges Verbinden des Halterahmens mit dem Träger und/oder
- integrales Herstellen des Halterahmens und des Trägers und/oder
- integrales Anformen des Halterahmens an dem Träger.

15. Verfahren zum Fixieren eines flächigen Funktionselements mit einem Adapter, insbesondere nach einem der Ansprüche 1 bis 9, umfassend
- Anordnen eines flächigen Funktionselements an einer stirnseitigen Auflagefläche eines Trägers,
- Mechanisches Fixieren des flächigen membranartigen Funktionselements an der stirnseitigen Auflagefläche des Trägers durch Bewegen eines beweglich mit einem einen stirnseitigen Endbereich des Trägers zumindest teilweise umgebenden Halterahmen verbundenen Verriegelungsrahmens des Adapters in eine Schließstellung und formschlüssiges Arretieren des Verriegelungsrahmens an dem Halterahmen in der Schließstellung.
